(19) Europäisches Patentamt
European Patent Office
Office européen des brevets

(11) Publication number: **0 174 114**
**A1**

(12) # EUROPEAN PATENT APPLICATION

(21) Application number: 85305731.3

(22) Date of filing: 13.08.85

(51) Int. Cl.⁴: C 07 F 15/00
A 61 K 31/28

(30) Priority: 14.08.84 US 640632

(43) Date of publication of application: 12.03.86 Bulletin 86/11

(84) Designated Contracting States: AT BE CH DE FR GB IT LI LU NL SE

(71) Applicant: ENGELHARD CORPORATION
70 Wood Avenue South CN 770
Iselin New Jersey 08830(US)

(72) Inventor: Amundsen, Alan R.
12 Meadowbrook Drive
Somerville New Jersey(US)

(72) Inventor: Hollis, Leslie S.
33 Guilford Lane
Mercerville New Jersey(US)

(72) Inventor: Stern, Eric W.
234 Oak Tree Road
Mountainside New Jersey(US)

(74) Representative: Fisher, Adrian John et al,
Carpmaels & Ransford 43 Bloomsbury Square
London WC1A 2RA(GB)

(54) **Platinum complexes of ascorbic acid and ascorbic acid analogs.**

(57) Compounds of the formula:

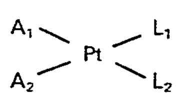

are disclosed as having anti-tumour activity. Pt is in valence state II; $A_1$ and $A_2$ are ammonia or aliphatic amine ligands; or $A_1$ and $A_2$ are amine nitrogen atoms of a chelating diamine ligand; $L_1$ is a carbon bound ligand which may be ascorbate or an ascorbate analogue such as tetramethylreductate; $L_2$ is selected from the same group of ligands as $L_1$ bound through an oxygen atom, or $L_2$ is OH, $H_2O$, halo, alkylcarboxy, nitrate, sulfate or phosphate.

Methods for the separation of individual isomers are also disclosed.

./...

EP 0 174 114 A1

Croydon Printing Company Ltd

FIG. 1—IR SPECTRUM OF (Pt (CIS-DACH) (ASCORBATE) (ISOMER V))

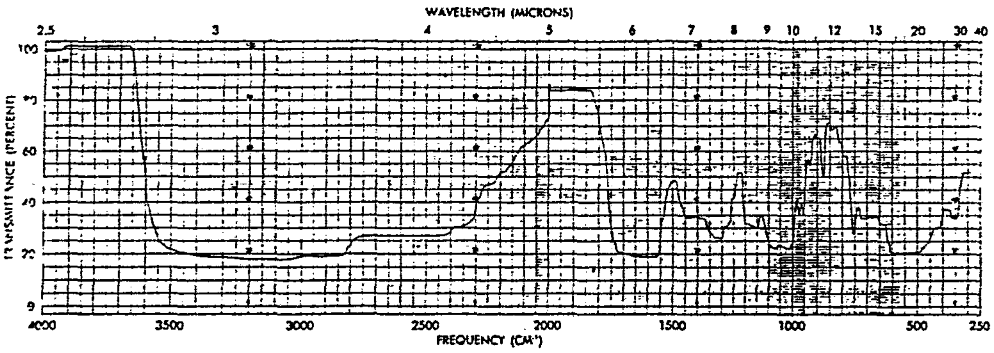

# PLATINUM COMPLEXES OF ASCORBIC ACID AND ASCORBIC ACID ANALOGS

This invention relates to platinum(II) complexes of ascorbic acid and, in particular, the synthesis of specific platinum complexes of ascorbic acid and ascorbic acid analogs. More specifically, the invention is concerned with the synthesis of structurally unique platinum complexes which exhibit pronounced activity against malignant tumors in animals.

## Brief Description of the Drawings

Fig. 1 is a reproduction of the infrared spectrum of [Pt(cis-dach)(ascorbate)](isomer VI) of Ex. 3.

Fig. 2 is a reproduction of the infrared spectrum of [Pt(cis-dach)(ascorbate)](isomer VII) of Ex. 3.

Fig. 3 is a reproduction of the infrared spectrum of [Pt(R,R-trans-dach)(ascorbate)] of Ex. 4.

Fig. 4 is a reproduction of the infrared spectrum of [Pt(S,S-trans-dach)(ascorbate)] of Ex. 4.

Fig. 5 is a reproduction of the $^{13}C$ NMR spectrum of [Pt(cis-dach)$(ascorbate)_2$] of Ex. 5.

Fig. 6 is a reproduction of the infrared spectrum of [Pt(cis-dach)$(ascorbate)_2$] of Ex. 5.

Fig. 7 is a reproduction of the infrared spectrum of cis-[Pt$(NH_3)_2$(ascorbate)] of Ex. 7.

Fig. 8 is a reproduction of the infrared spectrum of [Pt(trans-dach)(tetramethylreductate)] of Ex. 8.

## Background

Within the past ten years, transition metal complexes have become valuable agents in the clinical treatment of human cancer. Since "cisplatin", (cis-[Pt$(NH_3)_2Cl_2$]), was approved by the FDA for treatment of human malignancies, metal complexes have assumed an expanding role in the field of medicine. The widespread success that cisplatin is finding in the clinical

treatment of testicular and ovarian cancers has stimulated research in the area of inorganic-based anticancer drugs.

Recent advances in the clinic have improved the effectiveness of cisplatin and have made it one of the most promising chemotherapeutic agents that is presently available to the patient. Optimized drug delivery methods, such as prehydration and saline infusion, have been developed to minimize the adverse toxicological effects of the drug. Progress also has been made through recent efforts to expand the scope of cisplatin treatable cancers. A variety of forms of cancer are now being treated with cisplatin and new combination chemotherapy regimens are finding success in a number of clinical programs.

While cisplatin has proven successful in many cases, there are some types of cancer that remain unresponsive to treatment with the drug. This factor, coupled with the toxic side effects of the drug, has led to new synthetic research efforts which are directed toward the development of second generation platinum coordination complexes that exhibit a different spectrum of activity as well as decreased toxicity and improved solubility.

One possible approach to improvements in drug activity or decreases in toxicity as well as improved properties such as solubility and solution stability is synthesis of compounds containing platinum bound to biologically active molecules thus providing the possibility of new routes for cellular entry or delivery of the drug to the active site.

Among the numerous new platinum coordination complexes which are being developed for antitumor activity, one group of new compounds that has shown good activity in a variety of animal tumor screens are the diamineplatinum ascorbates developed at Engelhard Corporation and which are described in U.S. Patents 4,457,926 and 4,462,998.

The platinum(II) amine ascorbate complexes of these above-identified applications possess the following general formula:

$$cis\text{-}[Pt(II)A_2(X)_m(OH)_n] \quad (I)$$

wherein Pt is in valence state II and is coordinated to A in a cis configuration, A is ammonia ($NH_3$) or a monodentate alkylamine ligand, $A_2$ is a bidentate diamine ligand, X is the ascorbate moiety, m has a value of from 1 to 2, n has a value of from 0 to 1 and the sum of m and n is not greater than 2. The subscripts m and n may have fractional values between their respective limits in which event formula (I) represents a mixture of individual complexes.

The ascorbate moiety is derived from ascorbic acid as represented by the formula:

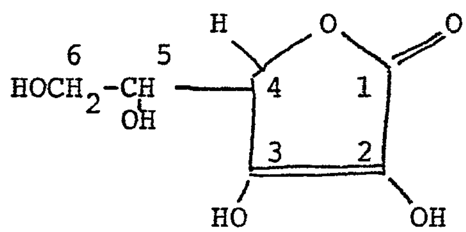

(II)

wherein carbons 4 and 5 are optically active. Accordingly, the ascorbic acid molecule may exist as any one of four optical isomers or as a mixture of same. The ascorbate moiety of the platinum(II) complex can thus be derived from any one of said individual isomers or from a mixture of any two or more of said isomers.

The platinum(II) ascorbate complexes are prepared by contacting in aqueous medium an ascorbate salt with an amine platinum(II) "diaquo" salt represented by the formula:

$$cis\text{-}[PtA_2(H_2O)_2]^{+2}(Z^{-(2-u)})_{1+u}$$

wherein A and $A_2$ are as defined above, Z is an inorganic anion, and u is a number having a value of 0 or 1. Suitable anions are those which are stable in acid media; they include, for example, the sulfate, nitrate and

perchlorate anions although nitrate is preferred. Anions having a greater complexing ability than water or ascorbate, such as chloride, bromide and iodide are not suitable.

The "diaquo" salt is formed from the stoichiometric reaction of a cis-diamineplatinum(II) dichloride with a silver salt, preferably silver nitrate, in an aqueous medium at room temperature.

Although the platinum amine ascorbate complexes formed by contacting an ascorbate salt with the amine platinum "diaquo" salt described above showed pronounced activity against malignant tumors in animals and high solubility in water with low toxicity, the platinum amine ascorbate complexes were found to comprise a mixture of platinum amine ascorbate compounds which had not been separated or characterized to determine their exact chemical structure.

## SUMMARY OF THE INVENTION

In accordance with the present invention, the individual platinum amine ascorbate complexes which are formed as a mixture in the preparative methods disclosed in U.S. Patents 4,457,926 and 4,462,998 as described above are isolated and characterized, and direct routes of synthesis provided.

In addition, analgous materials containing the key structural features of these materials are described.

The general formulation for this class of platinum compounds can be written as follows:

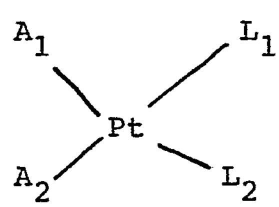

(III)

wherein $A_1$ and $A_2$ are ammonia or aliphatic amine ligands or $A_1$ and $A_2$, taken together, are a chelating diamine ligand.

The monodentate amine ligands $A_1$ and $A_2$ may be the same or different and can be represented by the formula $RNH_2$ and include ammonia ($NH_3$) and monoalkyl amines having up to 6 carbons in the alkyl group as, for example, methylamine, ethylamine, propylamine, isopropylamine, hexylamine and the like. Alkylamines having up to 3 carbon atoms are preferred. The alkyl group, R, also may contain substituents such as hydroxy, $C_1$-$C_4$ alkoxy, halo, carboxy, $C_1$-$C_4$ alkylcarboxy, or $C_1$-$C_4$ alkoxycarbonyl.

The chelating bidentate diamine ligands represented by $A_1$ and $A_2$ taken together are those of following formula (IV):

$$\begin{array}{c} \quad R^1 \;\; R^2 \\ \quad | \quad\; | \\ H_2N-CH-CH-NH_2 \end{array} \qquad (IV)$$

wherein each of $R^1$ and $R^2$, taken separately, represent hydrogen or lower alkyl and $R^1$ and $R^2$, taken together, afford a 1,2-diaminocycloalkane ligand containing from about 4-8 nuclear carbon atoms as, for example, 1,2-diaminocyclopentane, 1,2-diaminocyclohexane and 1,2-diaminocycloheptane which may optionally be substituted on the nuclear carbons by one or more linear or branched chain lower alkyl groups. By the use of the term "lower alkyl" is meant a linear or branched chain alkyl group of from 1-6 carbons and, preferably, from 1-3 carbons such as methyl, ethyl, or propyl. Preferred diamine ligands (IV) are those in which at least one of said $R^1$ and $R^2$ radicals represent hydrogen as, for example, ethylenediamine and propylene-1,2-diamine. Also preferred are diamines wherein $R^1$ and $R^2$, taken together, form a 1,2-diaminocyclohexane moiety. $R^1$ and $R^2$ also may represent such groups as hydroxy, $C_1$-$C_4$ alkoxy, halo, carboxy, $C_1$-$C_4$ alkylcarboxy, or $C_1$-$C_4$ alkoxycarbonyl or alkyl or cycloalkyl groups as defined above carrying these groups as substituents.

$L_1$ comprises the carbon bound species below:

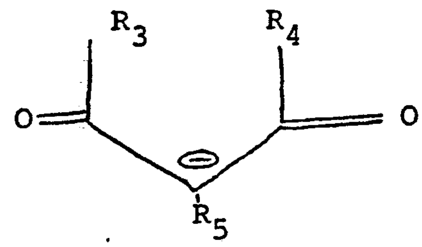

wherein $R_3$ and $R_4$ may be the same or different and may include hydrogen, $C_1$-$C_6$ alkyl, or $C_1$-$C_6$ alkoxy, or $R_3$ and $R_4$ together form a four to seven membered alicyclic or heterocyclic ring. These alkyl, or alkoxy groups or four to seven membered rings, may carry substituents including hydroxy, hydroxyalkyl ($C_1$-$C_4$), $C_1$-$C_4$ alkoxy, $C_1$-$C_4$ alkoxyalkyl ($C_1$-$C_4$), halo, carboxy, $C_1$-$C_4$ alkylcarboxy, $C_1$-$C_4$ alkylcarboxyalkyl ($C_1$-$C_4$), $C_1$-$C_4$ alkoxylcarbonyl, or $C_1$-$C_4$ alkoxycarbonylalkyl ($C_1$-$C_4$).

$R_5$ may be hydrogen, hydroxy, $C_1$-$C_4$ alkyl, $C_1$-$C_4$ alkoxy or phenyl.

$L_2$ may be the same ligand as $L_1$, bound preferably through an oxygen atom or may be a ligand selected from the same group of species as $L_1$, bound preferably through an oxygen atom. $L_2$ may also be OH, $H_2O$, dimethylsulfoxide (DMSO), halo, alkylcarboxy, or nitrate, sulfate, or phosphate.

When $L_2$ is an uncharged ligand such as either $H_2O$ or dimethyl sulfoxide (DMSO), the complex is charged and therefore, must exist with a counter ion. While any suitable counter ion may be employed, counter ions such as nitrate, perchlorate, acetate phosphate and sulfate

This definition includes the ascorbates, where $R_3$ and $R_4$ together comprise the following:

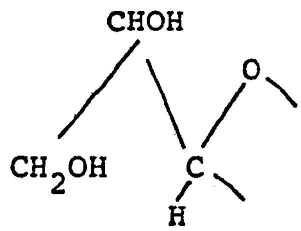

(Va)

and $R_5$ = OH. It also includes the other optical isomers of the ascorbates, including the isoascorbates, as well as ascorbate analogs such as tetramethylreductate:

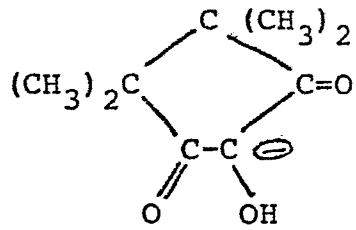

(Vb)

This definition also includes the beta-diketonates when $R_5$=H:

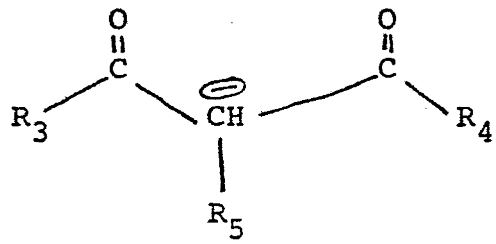

(Vc)

and the esters of malonic acid or substituted malonic acids when $R_3$ and $R_4$ (V) are $C_1$-$C_6$ alkoxy or substituted $C_1$-$C_6$ alkoxy.

As is apparent from the above description, $L_1$ may be present in either a 1:1 or 2:1 ratio with platinum. In the former case, the ligand may form a chelate, with binding through carbon and oxygen. In the latter case, the two ligands may be bound through carbon and oxygen respectively. Both types of complexes are present in Pt(diamine)(ascorbate) mixtures and tests indicate that both show antitumor activity in animals.

## DETAILED DESCRIPTION OF THE INVENTION

The diamineplatinum ascorbates as set forth in formula (I), the preparation of which is described in U.S. Patents 4,457,926 and 4,462,998, have been found to contain a variety of platinum(II) complexes. The exact number and structural characteristics of these complexes were unknown at the time of preparation.

It has now been found that the individual platinum(II) ascorbate complexes which are formed can be characterized by structural formulas such as VI, VII and VIII, (Pt:ascorbate=1:1), and corresponding structures in which Pt:ascorbate=1:2.

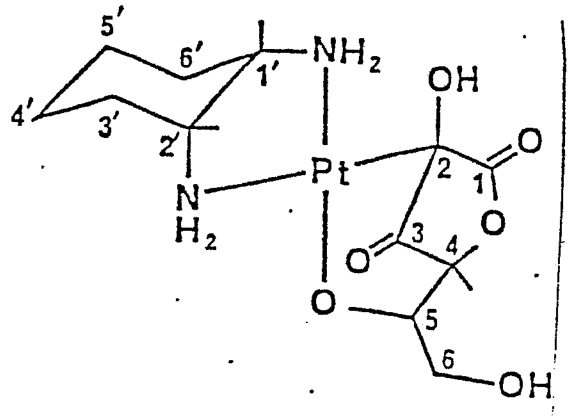

(VI)

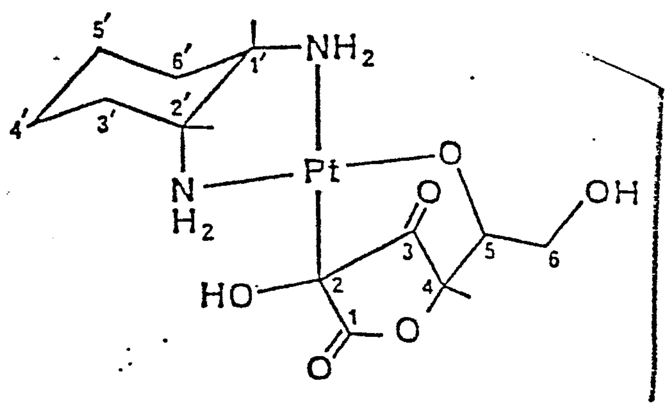

(VII)

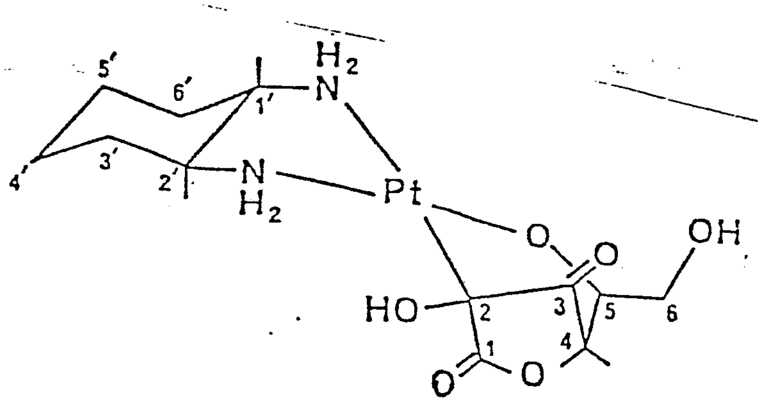

(VIII)

More specifically, studies of diaminocyclohexaneplatinum(II) ascorbate as prepared by methods disclosed in U.S. Patents 4,457,926 and 4,462,998 show that up to eight separate components corresponding to the general formulation (I) may be formed in the crude product. A major component has been identified as [Pt(dach)-(ascorbate)], as illustrated by structural formulas VI, VII, and VIII. Thus, referring to these structural formulas, four isomeric forms of this complex are produced. These isomeric forms can be explained in terms of the geometry of the dach ligand. The ascorbate ligand is attached to the 'Pt(dach)' moiety through carbon atom C2 and oxygen atom O5. Since the Pt(cis-dach) portion of the molecule is asymmetric with respect to rotation about the axis that bisects the N1-Pt-N2 angle, two possible isomers result when the chelating ascorbate is attached to the Pt(cis-dach) unit. For example, the complex that results when C2 is attached trans to N1 (VII) is not equivalent to the complex that results when C2 is attached trans to N2(VI). These two forms of Pt(cis-dach)(ascorbate) are different isomers and consequently have different chemical properties.

The [Pt(trans-dach)(ascorbate)] (VIII) analog also forms two isomers. Since the trans-dach portion of the molecule exists in the two enantiomeric forms (S,S and R,R) and because ascorbate is itself optically active, [Pt(trans-dach)(ascorbate)] exists in two diasteromeric forms. Because the 'Pt(trans-dach)' moiety is symmetric with respect to the axis bisecting the $N_1$-Pt-$N_2$ angle, isomerism of the type present in the cis-dach complex is absent.

Additionally, the 'Pt(dach)(ascorbate)' product comprises complexes containing two ascorbate ligands per platinum in which one ascorbate ligand is attached to platinum via a carbon atom and the other through an oxygen atom.

Again, as in the case of [Pt(dach)(ascorbate)] the isomerisms and asymmetry of the dach ligand results in four isomers of [Pt(dach)(ascorbate)$_2$]. As described above, two of these arise from the non-equivalence of the nitrogen atoms in the 'Pt(cis-dach)' moiety and two from the two enantiometric forms of trans-dach.

It should be understood that the number of possible components in the compounds which are the subject of this invention depends on the nature of the A and L ligands in formula III. Thus, for example, in the case of Pt(diamine)(ascorbate) complexes in which the two nitrogens are equivalent and in which the A groups contain no centers of asymmetry, such as in [Pt$(NH_3)_2$(ascorbate)$_n$], n=1 or 2, the number of possible components is decreased to two.

Similarly, the number of possible components will vary in compositions in which $L_1$ and $L_2$ (III) are different.

The complexes of this invention are useful in tumor chemotherapy having been found active against malignant tumors in animals as, for example, Sarcoma 180 ascites tumors in mammals such as mice. The anti-tumor effect exhibited by the subject complexes may also extend to other sarcomas, lymphoid leukemias and to such other tumors as the following: lymphosarcoma, myelocytic leukemia, malignant lymphoma, squamous cell carcinoma, adenocarcinoma, scirrhous carcinoma, malignant melanoma, seminoma, teratoma, choriocarcinoma, embryonalcarcinoma, cystadenocarcinoma, endometriocarcinoma or neuroblastoma and the like. In addition, said complexes may be useful as anti-viral, anti-inflammatory, anti-bacterial and anti-parasitic agents.

It has been found that when the chelated (Pt:ascorbate = 1:1) complexes of this invention are

contacted with an aqueous acid solution, the resulting solution has enhanced activity against Sarcoma 180 ascites tumors in mammals in comparison with the unacidified complex. While any suitable acid may be employed, it is presently preferred to use nitric acid, perchloric acid, acetic acid, sulfuric acid, ascorbic acid or mixtures thereof. A combination of nitric acid and ascorbic acid is particularly preferred at present.

Compare, for example, the activity screening data in Table III for Pt(R,R-trans-dach) ascorbate and the acid treated Pt(R,R-trans-dach) ascorbate (Ex. 12). Similarly, Pt(ethylenediamine)(ascorbate), which is acid treated (Ex. 11) exhibits enhanced activity when compared with its unacidified form (Ex. 10).

As a result of this enhanced activity from the acid treatment, it may be desirable to administer the relatively insoluble complexes orally, allowing the stomach acids to act on the complexes so as to enhance their activity.

The complexes of this invention may be administered parenterally or orally in admixture with a non-toxic pharmacologically acceptable inert carrier or diluent in any of the usual pharmaceutical forms. These include

solid and liquid oral unit dosage forms such as tablets, capsules, powders, and suspensions or solutions for subcutaneous, intramuscular, intravenous or intra-arterial injection. The term "unit dosage" refers to physically discrete units which may be administered in single or multiple dosages each containing a predetermined quantity of the active ingredient in association with the required diluent, carrier or vehicle. The quantity of active ingredient is the amount of complex needed to produce the desired therapeutic effect.

A typical unit dosage consists essentially of from about 10-1,500 mg of active ingredient; however, the form in which said ingredient is administered and the frequency of administration is usually determinative of its concentration. Thus, for example, oral unit dosage forms containing 5-1,500 mg of active ingredient may be administered one or more times per day depending upon the severity of the tumor which is sought to be treated and the condition of the host animal. By contrast, parenteral administration generally requires from about 10-400 mg of the active ingredient per unit dosage administered as a daily dose or as a fraction thereof depending upon whether the regimen calls for administration once, twice, three or four times daily.

By contrast to the "unit dosage," the effective dose is that total dosage which is needed to achieve the desired anti-tumor effect. In general, this dosage lies within the range of from about 15-3,800 mg of the active ingredient per kg. of body weight of the host animal (i.e., mg/kg). A preferred concentration lies within the range of from about 50-1,800 mg/kg. For oral administration an effective dose of 100-3,800 mg/kg has been found to be most suitable, whereas, in the case of parenteral administration it is usually advisable to employ from about 20-1,400 mg/kg. These dosages are well below the toxic or lethal does and they may be varied over a

wide range for the symptomatic adjustment of the dosage to the patient which is being treated.

The preferred compositions for oral administration are tablets in which the active ingredient is present in quantities of 5-1,500 mg in a pharmaceutically acceptable orally ingestible solid carrier. If desired, the composition may also contain flavors, binders, lubricants and other excipients known in the art.

An alternative oral mode is the soft gelatin capsule. Such a composition may also contain from 5-1,500 mg by weight of active ingredient dissolved or suspended in vegetable oil, peanut oil, alcohol or glycerine and the like.

A hard or dry-filled capsule may be prepared by admixing 25-500 mg of active ingredient with suitable excipients such as lactose and magnesium stearate and putting the mixture into a pre-formed and suitably sized gelatin sheath.

The complexes of this invention may also be formulated as liquid solutions or suspensions or as a dry powder for addition to foods, drinking water, fruit juice or other potable liquids.

Tablets are prepared by mixing a complex of this invention, in suitably comminuted or powdered form, with a diluent or base such as starch, sucrose, kaolin or dicalcium phosphate and the like. The resulting mixture can be granulated by wetting with a binder such as a syrup, starch (paste), acacia mucilage or solutions of cellulosic or polymeric materials, whereafter, the wetted mixture is forced through a screen. As an alternative to granulating, the powdered mixture can be run through a tablet machine and any slugs which are imperfect may be broken into granules. The granules are lubricated to prevent sticking to the tablet-forming dies via the addition of stearic acid, a stearate salt, talc or mineral oil and the lubricated mixture is then compressed into tablets. The

complexes of this invention can also be combined with free flowing inert carriers and then be subjected to compression so as to form tablets without going through the granulating or slugging steps. A protective coating or sealing coat of shellac, sugar or polymeric material and a polished coating of wax can also be provided. Dyestuffs may be added to distinguish different unit dosages.

In this invention the term "pharmacologically acceptable inert carrier or diluent" denotes a non-toxic substance which, when mixed with the active ingredient, renders it more suitable for administration. Compositions intended for oral administration may include such carriers or diluents as glucose, lactose, sodium or potassium ascorbate, sucrose, corn starch, potato starch, sodium carboxymethyl cellulose, ethyl cellulose, cellulose acetate, powdered gum tragacanth, gelatin, alginic acid, agar, stearic acid or the sodium, calcium and magnesium salts of stearic acid, sodium lauryl sulfate, polyvinylpyrrolidone, sodium citrate, calcium carbonate and di-calcium phosphate. Said compositions may also contain non-toxic adjuvants and modifiers such as dyes, buffering agents, preservatives, surfactants, emulsifiers, flavoring agents or biocides and the like.

The following embodiments illustrate the preparation of representative unit dosage forms.

<u>Compressed Tablet</u>

| | |
|---|---|
| [Pt(cis-dach)(ascorbate)] (VI) | 400 mg |
| Sodium Ascorbate | 200 mg |
| Niacinamide | 50 mg |
| Calcium Pantothenate | 20 mg |
| Magnesium Sulfate | 50 mg |
| Zinc Sulfate | 50 mg |
| Magnesium Stearate | 10 mg |
| | 780 mg |

The Pt(II) complex, sodium ascorbate, niacinamide, calcium pantothenate, magnesium sulfate, zinc sulfate and

magnesium stearate (5.0 mg) are mixed and compressed into slugs. The slugs are then broken into granules and sifted through an 8 mesh screen. Additional magnesium stearate (5.0 mg) is added and the mixture is then compressed into tablets suitable for oral administration.

Soft Gelatin Capsule

A soft elastic gelatin capsule is filled with the following ingredients:

| | |
|---|---|
| [Pt(cis-dach)(ascorbate)] (VII) | 400 mg |
| Wheat germ oil | 50 mg |
| Sunflower seed oil | 100 mg |
| | 550 mg |

The Pt(II) complex and wheat germ oil are mixed with sunflower seed oil and the resulting mixture is poured into gelatin capsules suitable for oral administration. An alternative embodiment provides for substituting sunflower seed oil and wheat germ oil with equal amounts of peanut oil to obtain an otherwise similar capsule which is also suitable for oral administration.

Dry Filled Capsule

A hard dry-filled capsule may be prepared from the following ingredients:

| | |
|---|---|
| [Pt(trans-dach)(ascorbate)] (VIII) | 200 mg |
| Niacinamide | 50 mg |
| Calcium Pantothenate | 10 mg |
| Sodium Ascorbate | 150 mg |
| | 410 mg |

The Pt(II) complex is reduced to a No. 60 powder. Niacinamide, calcium pantothenate and sodium ascorbate are passed through a No. 60 bolting cloth and these ingredients are added to the Pt(II) complex. This combination of ingredients is mixed for 10 minutes and then poured into a No. 3 size gelatin capsule.

Dry Powder

The following composition illustrates a representative dosage in dry powder form. In this embodiment the active ingredient is water soluble and it is combined with up to 60% by weight of a suitable flavoring agent. All quantities are in a weight-percent relationship.

| | |
|---|---|
| [Pt(cis-dach)(ascorbate)$_2$] | 25-90% |
| Flavoring Agent | 10-60% |
| Preservative | 0.1% |

The Pt(II) complex, flavoring agent and preservative are thoroughly blended to afford a homogeneous dry powder which is readily soluble in water. The resulting formulation may be used as a food additive or it may be blended with other therapeutic agents to afford combination-type medicinals. Alternatively, said powder may be dissolved in a pharmacologically acceptable diluent to afford a solution which is suitable for oral administration.

Compositions intended for parenteral administration may include such diluents and carriers as water-miscible solvents as, for example, sesame oil, groundnut oil, aqueous propylene glycol and a solution of sodium riboflavin. Typical of said compositions are solutions which contain the active ingredient in sterile form. An embodiment illustrating a dosage form suitable for intravenous injection is set forth below.

Parenteral Solution

Injectable solutions can be formulated by mixing an ampoule of active ingredient with an ampoule of sterile diluent:

| | |
|---|---|
| Ampoule: cis-Pt$(NH_3)_2$(ascorbate) | 300 mg |
| Ampoule: Sterile Water(Diluent for Injection) | 2 cc |

The Pt(II) complex and water are mixed thoroughly immediately prior to administration. If desired, one or

more other active ingredients may be added to provide an injectable solution having enhanced therapeutic activity.

Method of Preparation

The compounds of this invention are prepared from platinum complexes of the type:

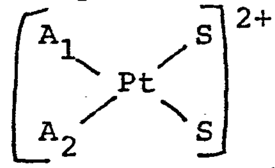 or 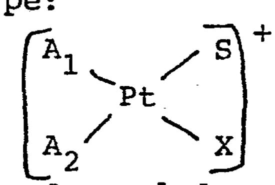

where S is water or an alcohol, $A_1$ and $A_2$ are defined above (III), and X is a halide.

The platinum complex is usually obtained by reacting $[PtA_1A_2X_2]$ X = halide, with one or two moles of a silver salt, preferably $AgNO_3$ in the solvent, S. The insoluble silver halide (AgX) formed is removed leaving, in this case, $[PtA_1A_2S_2](NO_3)_2$ or $[PtA_1A_2SX](NO_3)$ in solution.

The ligands $L_1$ and $L_2$ are as defined above and are either used in the form of alkali metal salts (e.g., sodium ascorbate or potassium ascorbate) or else the acid forms are reacted with or without one half or more equivalents of an alkali metal hydroxide.

The platinum complex as defined above, $L_1$ and $L_2$, are reacted in an aqueous or alcoholic medium. The molar ratio of $L_1$ and $L_2$ to Pt may range from 1:1 to as high as 10:1 but is preferably between 1:1 and 3:1. The concentration of the reactants is not extremely critical and may range from 0.01 to 1.0 molar; the preferred concentration is approximately 0.2 molar. $L_1$ and $L_2$ may be added to the platinum solution simultaneously or sequentially with $L_2$ following $L_1$ by as long as 24 hours. In the case where $L_2$=X, $PtA_1A_2L_1L_2$ may be prepared by reacting $L_1$ with $[PtA_1A_2XS]^+$. In the case where $L_2$=$H_2O$ or OH, $PtA_1A_2L_1L_2$ may be prepared by the reaction of $L_1$ with $[PtA_1A_2(H_2O)_2]^{2+}$.

The reaction mixture is stirred at ambient temperature for a period of time to facilitate the

reaction. If desired, temperatures above or below ambient temperatures as, for example, from about 0°C to 60°C, may be employed. The reaction may be conducted over a period of several minutes to 72 hours.

During this time a precipitate may form, which is then collected. In other cases the product may be precipitated from solution with a non-solvent or obtained by removal of solvent from the reaction solution by evaporation.

The product as thus obtained may contain a single compound as defined previously or may contain a mixture of such compounds, with or without the presence of unreacted starting material, non-Pt and degradation products. In the latter case, the desired products may be obtained in purified form by recrystallization from suitable solvents or via chromatographic techniques.

Examples 1-21, infra, illustrate the methods by which the compounds of this invention may be obtained and Example 22 describes the protocol used to evaluate their efficacy in mice. However, said examples are illustrative only and this invention should not be construed as being limited thereto because it will be apparent to one of ordinary skill that obvious modifications may be effected and functionally equivalent reagents may be substituted for those recited therein without departing from the spirit or scope of this invention.

## Example 1

### Separation of cis-dach and trans-dach

The resolution of the two isomeric forms of diaminocyclohexane can be accomplished using a published method (R. Saito, Y. Kidani, Chem. Lett, 123(1976)). The isomeric dach mixture is reacted with $NiCl_2.6H_2O$ in methanol. The resulting nickel complexes, $Ni(cis\text{-}dach)_2Cl_2$ and $Ni(trans\text{-}dach)_2Cl_2.2H_2O$ differ in solubility and can be easily separated by filtration. The separated nickel complexes are then decomposed by acid and the dach

isomers obtained by neutralization and extraction and purified by vacuum distillation.

The resulting cis-dach and trans-dach were converted to [Pt(cis-dach)$I_2$] and [Pt(trans-dach)$I_2$] using a published method (S.C. Dhara, Indian J. Chem, 8, 143 (1970)).

Example 2

'Pt(cis-dach)(ascorbate)' and 'Pt(trans-dach)(ascorbate)' Mixtures

Crude ascorbate mixtures were prepared according to the method of U.S. Patents 4,457,926 and 4,462,998, using [Pt(cis-dach)$I_2$] or [Pt(trans-dach)$I_2$], respectively, as the starting materials in the following reaction sequence:

(1) [Pt(dach)$I_2$]+$2AgNO_3$ ---> [Pt(dach)$(H_2O)_2$]$(NO_3)_2$+2AgI

(2) [Pt(dach)$(H_2O)_2$]$(NO_3)_2$+2Na(ascorbate) ---> 'Pt(dach)-(ascorbate)'

The freshly prepared 0.2M solutions of the diaquo complexes (eqn. 1) were mixed with 2 equivalents of ascorbic acid and 2 equivalents of NaOH. The resulting mixtures were stirred for 1-2 hours and the products were precipitated with ethanol. The crude ascorbate precipitates were collected by filtration and dried in vacuum. Alternatively crude ascorbate mixtures could be obtained by evaporating, the reaction solutions to dryness using a rotary evaporator.

Preparative HPLC separation of the 'Pt(cis-dach)(ascorbate)' and 'Pt(trans-dach)(ascorbate)' mixtures

The individual components of the Pt(cis-dach)-(ascorbate) and Pt(trans-dach)(ascorbate) mixtures were separated using a Waters HPLC equipped with a Whatman Magnum Partisil M7 ODS column (10mm X 50cm). Water was employed as the mobile phase; the flow rate was 4 ml/min. Detection was by UV absorbance at 254 nm. The output of

this detector was connected to a Siemens Model ES-100 Fraction Collector equipped with an automatic repeat injection feature. Fraction collection times were set based on the first of a series of injections; the fraction collector then automatically repeated the collection sequence for as many as 15 additional injections. Each injection contained ca. 100mg of product mixture in 2ml $H_2O$. The fractions were collected in vessels cooled with dry ice. The frozen fractions were evaporated to dryness using a lyophilizer.

In addition to the materials described below, fractions containing unreacted starting material, non-Pt compounds, and ascorbate degradation products were obtained. The quantities of the latter could be reduced by running the preparative reaction under an inert atmosphere.

Example 3

Pt(cis-dach(ascorbate).$3H_2O$ isomers (VI) and (VII)

A solution of freshly prepared [Pt(cis-dach)$(H_2O)_2$]$(NO_3)_2$ solution (0.2M, 50ml) was treated with 3.52g of ascorbic acid (ascorbic acid:Pt=2.0) and 1.6g of NaOH (NaOH:Pt=4.0). The resulting solution was stirred under nitrogen, at room temperature for 24 hrs. The precipitate that formed during this time was then removed by filtration, washed with water (20ml) and vacuum dryed. A yield of 3.12g (58%) of mixed isomers (VI) and (VII) was obtained.

A 1.5g sample of the mixture was dissolved by stirring in 23 ml of ethylene glycol and filtered to remove insolubles. The filtrate was treated with 50 ml of water and cooled to 4°C for 1 hour. The resulting white solid (isomer VII) was collected by filtration. The filtrate was poured into 500 ml of acetone and cooled to 0°C for 2-3 hours. The resulting precipitate (isomer VI) was then collected by filtration, washed with acetone and air dried. The separated fractions, 0.8g of isomer VII

and 0.5g of isomer VI, were found to be greater than 95% pure by HPLC.

Anal of isomer (VI) Calc. for $PtC_{12}H_{26}N_2O_9$: Pt, 36.30; C,26.82; H,4.88; N,15.21. Found: Pt, 36.41; C,26.64; H,4.92; N,5.13.

Anal of isomer (VII). Calc. for $PtC_{12}H_{26}N_2O_9$: Pt, 36.30; C,26.82; H,4.88; N,5.21. Found: Pt, 36.14; C,26.31; H,4.98; N,5.10.

X-ray diffraction studies on Pt(cis-dach)-(ascorbate) (VI) were undertaken using a colorless monoclinic plate with dimensions of 0.20mm X 0.03mm X 0.30mm and bounding faces of the forms {010}, {011} and {210}. The unit-cell parameters were determined from the setting angles of 25 randomly oriented reflections using a Nonius CAD-4F diffractometer. Isomer VI crystallizes in the monoclinic system (space group $P2_1$) with the following cell parameters: a = 6.425(1), b = 20.542(2), c = 6.662(1)Å; $\beta$=104.90(1)°. The measured density ($\rho_{obs}$ = 2.094g/$cm^3$) of the crystals gives a calculated molecular weight of 534.9 for VI, assuming 2 molecules/unit cell. The calculated molecular weight is 536.3 for [Pt(cis-dach)(ascorbate)].$3H_2O$.

The structure was determined by standard Patterson and Fourier methods using 1633 unique reflections collected on a CAD-4F diffractometer with Mo $K\alpha$ radiation. All calculations were performed on a DEC VAX-11/780 computer using the SHELX-76 x-ray programs. Refinement of the structure converged to a value of 0.020 for the discrepancy factor $R_1$, using isotropic thermal parameters for all atoms except Pt, which was refined anisotropically. The hydrogen atoms were refined with constraints using a common temperature factor.

The resulting structure clearly shows the ascorbate ligand bound to platinum through carbon (C2) and oxygen (O5).

$^{13}C$-NMR data for [Pt(cis-dach)(ascorbate)].$3H_2O$ (isomers VI and VII) are included in Table I. $^{195}Pt$-NMR data for these compounds are shown in Table II. Infrared spectra for isomers VI and VII are shown in Figures 1 and 2. These data support the conclusion that isomer VII also contains ascorbate bound through C2 and O5 and that these isomers are closely related.

Both isomers are highly stable in aqueous solution. In hot water they are slowly interconverted. A mixture of isomers VI and VII also may be separated by fractional crystallization from water.

The two isomers of [Pt(cis-dach) (ascorbate)] .$3H_2O$ also can be isolated from the 'Pt(cis-dach)-(ascorbate)' mixture of Example 2 by preparative HPLC.

Example 4

[Pt(R,R-trans-dach)(ascorbate)].$3H_2O$ and [Pt(S,S-trans-dach)(ascorbate).$2H_2O$]

[Pt(R,R-trans-dach)$I_2$](8.00g) and $AgNO_3$ (4.83 g, Ag:Pt = 2) were stirred in 100 ml water for 1 hr. After removal of the AgI by filtration, 2 equivalents of ascorbic acid (5.00g) and 1 equivalent of NaOH (0.57g) were added, the latter as a solution in 10 ml $H_2O$. The mixture was stirred under $N_2$ overnight. The resulting white precipitate was filtered yielding 2.65g crude product. One gram of this was recrystallized from 70 ml 25% aqueous methanol, filtering at 70 deg C and refrigerating the filtrate for 72 hr. The yield was 0.72g of [Pt(R,R-trans-dach)(ascorbate)].$3H_2O$. Anal. Calc. for $PtC_{12}H_{26}N_2O_9$:Pt,36.30; C,26.82; H,4.88; N,5.21. Found: Pt,36.16; C,26.21; H,4.96; N,4.98.

TABLE I

$^{13}C$ NMR DATA FOR Pt(dach)(ascorbate) AND cis-[Pt($NH_3$)$_2$(ascorbate)]

A. Ascorbate Resonances*

| Compound | C1 | C2 | C3 | C4 | C5 | C6 |
|---|---|---|---|---|---|---|
| cis-[Pt($NH_3$)$_2$(ascorbate)] | 199.4 | 66.5 | 176.0 | 80.9 | 85.2 | 64.9 |
| [Pt(cis-dach)(ascorbate)](Isomer VI) | 196.5 | 69.5 | 175.7 | 80.7 | 85.2 | 64.8 |
| [Pt(cis-dach)(ascorbate)](Isomer VII) | 199.5 | 69.1 | 174.6 | 80.5 | 85.8 | 64.8 |
| [Pt(R,R-trans-dach)(ascorbate)] | 198.7 | 69.1 | 175.6 | 80.7 | 85.8 | 64.9 |
| [Pt(S,S-trans-dach)(ascorbate)] | 198.0 | 69.5 | 175.4 | 80.5 | 85.5 | 64.8 |
| Ascorbic Acid - Free | 173.2 | 117.7 | 155.4 | 76.1 | 68.8 | 62.7 |
| Ave. Shift Upon Pt Binding (Free-Bound) | -25.2 | +48.9 | -20.1 | -4.6 | -16.7 | -2.1 |

B. Dach Resonances

| Compound | C4',5' | C3',6' | C1',2' |
|---|---|---|---|
| [Pt(cis-dach)(ascorbate)](Isomer VI) | 20.0<br>20.7 | 25.7<br>26.5 | 55.5<br>56.5 |
| [Pt(cis-dach)(ascorbate)](Isomer VII) | 20.1<br>20.5 | 25.7<br>26.4 | 55.4<br>56.6 |
| [Pt(R,R-trans-dach)(ascorbate)] | 24.0<br>24.1 | 31.9<br>32.6 | 59.2<br>61.1 |
| [Pt(S,S-trans-dach)(ascorbate)] | 24.2<br>24.2 | 32.6<br>31.9 | 61.4<br>59.5 |
| [Pt(cis-dach)($H_2O$)$_2$]$^{2+}$ | 20.0 | 25.3 | 58.8 |
| [Pt(trans-dach)($H_2O$)$_2$]$^{2+}$ | 24.9 | 32.4 | 64.1 |

* Chemical shifts are reported relative to $d_6$-DMSO at 39.5 ppm.

## TABLE II

### $^{195}Pt$ NMR DATA FOR $[Pt(amine)_2(ascorbate)]$ COMPLEXES

| Compound | Chemical Shift* (δ ppm) |
| --- | --- |
| cis-$[Pt(NH_3)_2(ascorbate)]$ | -2499 |
| [Pt(R,R-trans-dach)(ascorbate)] | -2558 |
| [Pt(S,S-trans-dach)(ascorbate)] | -2560 |
| [Pt(cis-dach)(ascorbate)]-(isomer VI) | -2524 |
| [Pt(cis-dach)(ascorbate)]-(isomer VII) | -2545 |
| $[Pt(cis\text{-}dach)(ascorbate)_2]$ | -2593<br>-2606 |
| cis-$[Pt(NH_3)_2(H_2O)_2]^{2+}$ | -1584† |
| $[Pt(cis\text{-}dach)(H_2O)_2]^{2+}$ | -1860† |
| $[Pt(trans\text{-}dach)(H_2O)_2]^{2+}$ | -1873† |

* Chemical shift measured relative to $K_2PtCl_4$ at -1627 ppm.
† at pH = 3.

[Pt(S,S-trans-dach)$I_2$] (5.00g) and $AgNO_3$ (3.02g, Ag:Pt=2.0) were stirred in 75 ml water at 45-50$^o$ C for 1 hr. After cooling, the AgI was removed by filtration and 2 equivalents ascorbic acid (3.13g), and 1 equivalent NaOH (0.35g) were added, the latter as a solution in 10 ml $H_2O$. This mixture was stirred overnight under $N_2$. It was filtered to remove a small trace of metallic impurity, then taken to dryness on a rotary evaporator, leaving a viscious amber oil. The oil was shaken in 100 ml ethanol in which it completely dissolved. This solution was added gradually to 600 ml acetone with stirring, and the mixture refrigerated overnight. The resulting white precipitate was filtered yielding 1.2g crude material containing three components. This was dissolved in 10 ml $H_2O$ and 1.2g gamma-alumina added. The solution was allowed to stand over the alumina for 72 hours. When the alumina was filtered off, crystals began to form in the filtrate. After refrigeration for 1 hour the cyrstals were collected. The yield was 0.3g of [Pt(S,S-trans-dach)(ascorbate)].

Anal Calc. for $PtC_{12}H_{24}N_2O_8$:Pt,37.56; C,27.75; H,4.66; N,5.39. Found:Pt, 37.41; C, 27.92; H,4.57; N,5.30.

Infrared spectra of [Pt(R,R-trans-dach)-(ascorbate)] and [Pt(S,S-trans-dach)(ascorbate)] appear in Figures 3 and 4.

These two isomers are diastereomers, having different physical and chemical properties. The R,R isomer is less than 1/10 as water soluble as the S,S isomer. An attempt to prepare a mixture of these diastereomers from racemic trans-dach resulted in precipitation of the R,R isomer only, with the S,S isomer remaining in solution. This affords a method of resolving racemic trans-dach into its R,R and S,S forms. As with the cis-dach complexes the isomers of [Pt(trans-dach)(ascorbate)] are highly water-stable.

Example 5

Pt(cis-dach)(ascorbate)$_2$ $\cdot 2H_2O$

A suspension of 5.63g [Pt(cis-dach)$I_2$] and 3.36g $AgNO_3$ in 100ml of methanol was stirred for 1.5 hours at 50°C. The resulting AgI was removed by filtration and 3.52g of ascorbic acid (ascorbic acid:Pt=2) and 0.48g of LiOH (LiOH:Pt=2) were added to the filtrate. The resulting solution was stirred for 24 hrs. at room temperature and then filtered to remove a grey-white precipitate. The filtrate was evaporated to an oil and mixed with 200 ml of isopropanol. The solid that formed (yield 1.5g) was removed by filtration. This solid was found by HPLC to contain mostly the desired product ( 60%). The yield was increased to 90% using the following re-precipitation procedure. A 700 mg sample of the solid was stirred in 5 ml of $H_2O$ and filtered to remove insolubles, ([Pt(cis-dach)(ascorbate)] isomers (VI) and (VII)). The filtrate was stirred with 80 ml of isopropanol and cooled to $0^oC$ for 1 hour. The resulting precipitate was removed by filtration. This sample (200 mg) was found to be 85-90% pure by HPLC. Further purification was accomplished by preparative HPLC using conditions similar to those in Example 2. Anal. Calc. for $PtC_{18}H_{32}N_2O_{14}$: Pt, 28.05; C,31.08; H, 4.64; N, 4.03. Found: Pt, 27.70; C, 30.90; H, 4.40; N, 3.99.

The $^{13}$C-NMR spectrum of this material is given in Figure 5. This suggests that the two ascorbate ligands are bound to Pt in a non-equivalent fashion. The most likely formulation is that [Pt(cis-dach)(ascorbate)$_2$] contains both a carbon bound (through C2) and an oxygen bound (through O3) ascorbate ligand.

The $^{195}$Pt-NMR data for [Pt(cis-dach)(ascorbate)$_2$] are shown in Table II. The presence of two absorbances suggests that two distinct species are present. This requires that the two ascorbate ligands are non-equivalent. The isomerism arises, as was the case

with the chelated species (Example 3), from the non-equivalence of the nitrogen atoms in the Pt(cis-dach) system.

The infrared spectrum of [Pt(cis-dach)(ascorbate)$_2$] appears in Figure 6.

Example 6

[Pt(trans-dach)(ascorbate)$_2$].$2H_2O$

Pt(trans-dach)$I_2$ (11.26g) and $AgNO_3$ (6.72g, Ag:-Pt=2.0) were suspended in 200 ml methanol and stirred at 55-60$^{o}$C for 1.5 hours. The resulting mixture was refrigerated for 30 min, and the AgI removed by filtration. Ascorbic acid (7.04g, ascorbic acid:Pt=2) was added, followed by a solution of NaOH (1.60g, NaOH:Pt=2) in 30 ml of methanol. The resulting solution was stored at room temperature for 2½ hours. A small quantity of dark solid was removed by filtration; the solution was then taken to dryness on a rotary evaporator. The resulting oil was separated via HPLC using a Whatman Magnum, M-7 ODS-3 50 cm. column employing repeated injections of 300 mg in 2.5 ml $H_2O$. Water was used as the mobile phase at a flow rate of 4 ml/min. Under these conditions the desired fraction eluted at 1300-1780 sec after injection. The fraction was frozen on collection; the solid product was obtained by lyophilization. The yield was 0.52 g Anal. Calc. for $PtC_{18}H_{32}N_2O_{14}$:Pt, 28.05; C, 31.08; H, 4.64; N 4.03, Found: Pt, 28.37; C, 30.83; H,4.72; N, 4.11.

This material actually contains a mixture of the S,S and R,R enantiomers of trans-dach. Although the resulting ascorbate complexes are diastereomers owing to the chirality of ascorbate ligand, they are not resolved by HPLC on a $C_{18}$ column.

Example 7

Cis-[Pt($NH_3$)$_2$(ascorbate)].$2H_2O$

The cis-diammine analog was prepared from the reaction of cis-[Pt($NH_3$)$_2$($H_2O$)$_2$]($NO_3$)$_2$ (180ml, 0.1M) with 2 equivalents of ascorbic acid and 2 equivalents NaOH, as described above. After stirring for 24 hrs, under $N_2$, the light green precipitate was removed by filtration and air dried (3.1g). The filtrate was reduced in volume to 30ml

and a second crop of product was collected by filtration. The total yield was 4.35g (54%). Anal Calc for $PtC_6H_{17}N_2O_8$: Pt, 44.31; C, 16.37; H, 3.89; N, 6.36. Found: Pt, 44.46; C, 16.27; H, 3.59; N, 6.40. $^{13}C$-NMR data are given in Table I and $^{195}Pt$-NMR data are given in Table II. Taken together, these data support ascorbate binding through C2 and O5 as is the case with [Pt(dach)-(ascorbate)] chelates. The infrared spectrum of cis-[Pt$(NH_3)_2$(ascorbate)] is given in Figure 7.

## Example 8

**[Pt(cis-dach)(isoascorbate)]**

The procedure used to prepare this analog is identical to that for [Pt(cis-dach)(ascorbate)] (isomers VI and VII) given in Example 3. The yield was 3.2g (59%). The product containing two components analogous to isomers VI and VII of [Pt(cis-dach)(ascorbate)].

## Example 9

**[Pt(trans-dach)(tetramethylreductate)]·$2.5H_2O$**

Pt(trans-dach)$_2I_2$(5.63g) and $AgNO_3$ (3.40 g, Ag:-Pt=2.0) were stirred in 60 ml water for one hr at 55$^o$ C. After cooling for ½ hour, the resulting AgI was removed by filtration and to the filtrate was added solid tetramethylreductic acid (3.48 g, tmra:Pt=2.0) and a solution of NaOH (0.4g, OH:Pt=1.0) in 10 ml of water. The resulting mixture was stirred for 3 hours and the grey-green precipitate which formed was filtered. It was dissolved in ca 200 ml methanol and filtered to remove metallic Pt. The methanol solution was then allowed to evaporate to dryness. The resulting solid was stirred with 25 ml methanol and filtered. The resulting off-white solid was washed with ether and air dried. The yield was 1.88g Anal Calc. for $PtC_{15}H_3N_2O_{5.5}$:Pt,37.34; C,34.48; H,5.98; N,5.36. Found: Pt, 37.33; C,34.46; H,6.06; N,5.39. The infrared spectrum of this compound is shown in Figure 8.

## Example 10

### Pt(ethylenediamine)(ascorbate)

Pt(en)$I_2$ (5.08 g, 10 mmoles) was suspended in 75 ml water and $AgNO_3$ (3.38 g, 20 mmoles) added. The mixture was warmed with stirring to 50°C. for 1 hr., then cooled, with stirring, to 25°C. The solid AgI was removed by filtration and washed with 25 ml water. The filtrate and washing were combined and deoxygenated by passing a stream of $N_2$ through the solution. With the $N_2$ flow maintained, ascorbic acid (3.52 g, 20 mmoles) was added followed by a solution of NaOH (0.8 g, 20 mmoles) in 6.5 ml water. The resulting solution was stoppered under $N_2$ and stirred at room temperature for 24 hr. The precipitate which formed was filtered, and vacuum dried to constant weight. The yield was (1.75 g, 41.0%).

## Example 11

### Pt(ethylenediamine)(ascorbate)

Pt(ethylenediamine) (ascorbate) 256 mg was suspended in a solution of 256 mg of ascorbic acid in 8 ml water. The pH of the resulting mixture was adjusted to 1.5 with 1N $HNO_3$, at which point the platinum complex dissolved. This solution was diluted serially to provide doses of 160 mg/kg and below for use in activity testing. In the activity testing, the results of which are shown in Example 19, Table III, all mice were injected within 30 minutes of the preparation of the initial solution.

Example 12

Acidified Pt(trans-R,R-dach)(ascorbate)

Pt(trans-R,R-dach)(ascorbate) (256 mg) was suspended in 8 ml water and ascorbic acid (256 mg) added. To this was added 2 ml 1N $HNO_3$, which was sufficient to dissolve the platinum complex. The resulting pH was 1.4. This solution was used in activity testing for a 640 mg/kg dose.. Lower doses were obtained by serial dilution of this solution with water. In the activity testing, the results of which are shown in Example 19, Table III, all mice were injected within 30 minutes of the preparation of the initial solution.

The following may be prepared using the general method given above or the variations of Examples 2-12.

| Example | $A_1$ | $A_2$ | $L_1$ | $L_2$ |
|---|---|---|---|---|
| 13 | $CH_3NH_2$ | $CH_3NH_2$ | ascorbate | |
| 14 | Ethylenediamine | | ascorbate | ascorbate |
| 15 | $NH_3$ | isopropylamine | isoascorbate | isoascorbate |
| 16 | 1,2-diaminocyclohexane | | 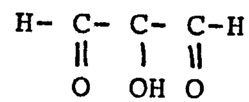 | $Cl^-$ |
| 17 | 4,5-dihydroxy-1,2-diamino cyclohexane | | 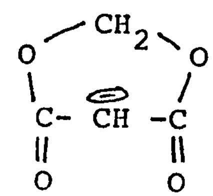 | $H_2O$ |
| 18 | Ethanolamine | Ethanolamine | Tetrametryl-reductate | $OH^-$ |
| 19 | $NH_3$ | $(HOCH_2)_3CNH_2$ | acetylacetonate | $Cl^-$ |
| 20 | 2-chloro-1,3-diamino-propane | | diethylmalonate | $NO_3^-$ |
| 21 | $(HOCH_2CH_2)NH$ | $(HOCH_2CH_2)_2NH$ | hexafluoracetyl-acetonate | $Br^-$ |

Example 22

Screening of compounds for antitumor activity against S180a

The various Pt ascorbate analogs were tested for antitumor activity against S180 ascites in female CFW Swiss mice by the following procedure.

CFW mice, averaging 20 g, are immediately inspected and placed in newly prepared cages. On day zero the mice are inoculated with 0.2 ml of a freshly prepared saline suspension (0.15 M NaCl) containing 1 x $10^7$ tumor cells/ml, or a total of 2 x $10^6$ cells. This inoculum is freshly prepared using "transfer" mice which have been injected with tumor cells the previous week. This inoculum is the end product of a series of steps which involve (1) the removal of the cells from the peritoneal cavity of the sacrificed transfer mouse, (2) alternate centrifugation-washing (2-3 times with cold saline) to remove occasional blood and other undesirable components, and finally (3) dilution (1:3) of the packed cell volume with saline (the final centrifugation being carried out at 1,000 rpm for 2 min). A cell count is made on a 2,000--fold dilution of this 1:3 suspension by means of a Coulter Counter. A final dilution to 1 x $10^7$ cells/ml is made based on the averaged count. On day 1, solutions of the test compounds are prepared and the mice injected, with each mouse of a set of six being injected with the same test compound at the same dose level. Also, on this day, two types of controls (6 mice/set) are employed: (1) Normal (1 set): 0.5 ml of the solvent medium used for the test compound, and (2) Positive control (1 set): a known anti-tumor agent (cis-[Pt$(NH_3)_2Cl_2$] in saline at 8 mg/kg), used to test the response to the biological system.

The effectiveness of a compound is measured in terms of the increase in life span (% ILS) of the test animals relative to the controls (calculated from the day of tumor inoculation, day zero). In order to standardize

the test data and permit intercomparisons to be made, the day of evaluation is arbitrarily taken as that day corresponding to twice the mean life-span (or average day of death) of the normal controls. This sets a practical upper limit of 100% on the ILS attainable. For calculation purposes, survivors on the day of evaluation are considered to have died on that day. The % ILS is formulated as:

$$\% \text{ ILS} = \left( \frac{\text{mean life-span of test mice}}{\text{mean life-span of control mice}} - 1 \right) \times 100\%$$

ILS values above 50% represent significant activity; those above 75% represent excellent activity.

A summary of the results is presented in Table III. All of the materials tested met the criterion for activity (50% ILS at one or more doses).

TABLE III

ANTITUMOR SCREENING DATA FOR VARIOUS COMPOUNDS

| Compound | Dose (mg/kg) | %ILS | 30-Day Survivors | Control %ILS | Control 30-Day Survivors |
|---|---|---|---|---|---|
| 'Pt(cis-dach)-(ascorbate)'-mixture (Ex.2)* | 5 | 12 | 0/6 | 98 | 6/6 |
| | 10 | 19 | 0/6 | | |
| | 20 | 39 | 0/6 | | |
| | 40 | 57 | 0/6 | | |
| | 80 | 74 | 4/6 | | |
| | 160 | 71 | 2/6 | | |
| 'Pt(trans-dach)-(ascorbate)' - mixture (Ex. 2)* | 5 | 28 | 0/6 | 98 | 6/6 |
| | 10 | 50 | 0/6 | | |
| | 20 | 89 | 4/6 | | |
| | 40 | 89 | 5/6 | | |
| | 80 | 51 | 1/6 | | |
| | 160 | -74 | 0/6 | | |
| [Pt(cis-dach)-(ascorbate)] (Isomer VI)* | 5 | 15 | 0/6 | 67 | 1/6 |
| | 10 | 23 | 0/6 | | |
| | 20 | 26 | 0/6 | | |
| | 40 | 22 | 0/6 | | |
| | 80 | 103 | 6/6 | | |
| | 160 | 99 | 4/6 | | |
| [[Pt(cis-dach)-(ascorbate)] (Isomer VI)* | 80 | 44 | 0/6 | 77 | 4/6 |
| | 160 | 77 | 3/6 | | |
| | 320 | 93 | 5/6 | | |
| [Pt(cis-dach)-(ascorbate)] (Isomer VII)* | 5 | 12 | 0/6 | 67 | 1/6 |
| | 10 | 20 | 0/6 | | |
| | 20 | 20 | 0/6 | | |
| | 40 | 43 | 0/6 | | |
| | 80 | 71 | 1/6 | | |
| | 160 | 98 | 5/6 | | |
| [Pt(cis-dach)-(ascorbate)] (Isomer VII)* | 80 | 79 | 2/6 | 77 | 4/6 |
| | 160 | 69 | 3/6 | | |
| | 320 | -59 | 0/6 | | |

* Administered in water.

TABLE III - Continued

ANTITUMOR SCREENING DATA FOR VARIOUS COMPOUNDS

| Compound | Dose (mg/kg) | %ILS | 30-Day Survivors | Control %ILS | Control 30-Day Survivors |
|---|---|---|---|---|---|
| [Pt(R,R-trans-dach)-(ascorbate)]* | 10 | 9 | 0/6 | 79 | 2/6 |
| | 20 | 6 | 0/6 | | |
| | 40 | 9 | 0/6 | | |
| | 80 | 15 | 0/6 | | |
| | 160 | 30 | 0/5 | | |
| | 320 | 55 | 3/6 | | |
| [Pt(R,R-trans-dach)-(ascorbate)]* | 80 | 7 | 0/6 | 76 | 0/6 |
| | 160 | 3 | 0/6 | | |
| | 320 | 65 | 3/6 | | |
| | 640 | 100 | 5/6 | | |
| | 800 | 72 | 5/6 | | |
| | 1280 | 100 | 6/6 | | |
| [Pt(cis-dach)-$(ascorbate)_2$]* | 10 | 21 | 0/6 | 67 | 3/6 |
| | 20 | 12 | 0/6 | | |
| | 40 | 29 | 0/6 | | |
| | 80 | 60 | 0/6 | | |
| | 160 | 97 | 5/6 | | |
| | 320 | 68 | 1/6 | | |
| cis[Pt$(NH_3)_2$-$(ascorbate)_2$]* | 10 | -2 | 0/6 | 86 | 2/6 |
| | 20 | 6 | 0/6 | | |
| | 40 | -6 | 0/6 | | |
| | 80 | -1 | 0/6 | | |
| | 160 | 57 | 1/6 | | |
| | 320 | 79 | 3/6 | | |
| [Pt(cis-dach)-(isoascorbate)]* | 10 | 9 | 0/6 | 98 | 3/6 |
| | 20 | 6 | 0/6 | | |
| | 40 | 6 | 0/6 | | |
| | 80 | 17 | 0/6 | | |
| | 160 | 4 | 0/6 | | |
| | 320 | 12 | 0/6 | | |
| [Pt(cis-dach)-(isoascorbate)]* | 160 | 10 | 0/6 | 67 | 3/6 |
| | 320 | 22 | 0/6 | | |
| | 640 | 70 | 2/6 | | |
| | 800 | 92 | 2/6 | | |
| | 1280 | 82 | 3/6 | | |
| | 1920 | 54 | 0/6 | | |

* Administered in water.

TABLE III - Continued

ANTITUMOR SCREENING DATA FOR VARIOUS COMPOUNDS

| Compound | Dose (mg/kg) | %ILS | 30-Day Survivors | Control %ILS | Control 30-Day Survirors |
|---|---|---|---|---|---|
| Pt(ethylene-diamine)(ascorbate)* (Ex. 10) | 10 | 4 | 0/6 | 94 | 5/6 |
| | 20 | 6 | 0/6 | | |
| | 40 | 22 | 0/6 | | |
| | 80 | -2 | 0/6 | | |
| | 160 | 10 | 0/6 | | |
| | 320 | 24 | 0/6 | | |
| Pt(ethylene-diamine)(ascorbate)+ (Ex. 11) | 5 | 3 | 0/6 | 94 | 5/6 |
| | 10 | 27 | 0/6 | | |
| | 20 | 45 | 0/6 | | |
| | 40 | 75 | 3/6 | | |
| | 80 | 93 | 3/6 | | |
| | 160 | 70 | 2/6 | | |
| Pt(trans-R,R-dach)(ascorbate) (Ex. 12)+ | 20 | 67 | 3/6 | 71 | 3/6 |
| | 40 | 83 | 3/6 | | |
| | 80 | 82 | 4/6 | | |
| | 160 | -75 | 0/6 | | |
| | 320 | -78 | 0/6 | | |
| | 640 | -94 | 0/6 | | |

* Administered in water.

+ Administered in $HNO_3$ ascorbic acid.

CLAIMS:

1. A cis-diamine platinum (II) compound having the formula:

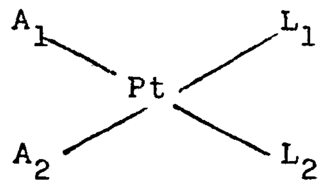

wherein Pt is in valence state II; $A_1$ and $A_2$ are ammonia or aliphatic amine ligands; or $A_1$ and $A_2$ are amine nitrogen atoms of a chelating diamine ligand; $L_1$ is a carbon bound ligand of the general formula:

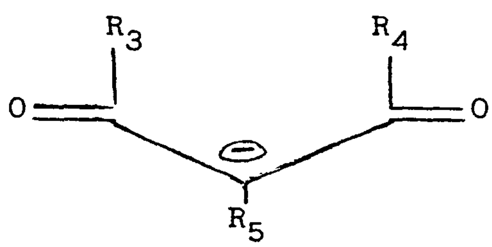

wherein $R_3$ and $R_4$ are the same or different and include hydrogen, $C_1$-$C_6$ alkyl, or $C_1$-$C_6$ alkoxy, or substituted derivatives thereof; or $R_3$ and $R_4$ together form a four to seven membered alicyclic or heterocyclic ring, or substituted derivatives thereof; $R_5$ is hydrogen, hydroxy, $C_1$-$C_4$ alkyl, $C_1$-$C_4$ alkoxy or phenyl; $L_2$ is selected from the same group of ligands as $L_1$ bound through an oxygen atom, or $L_2$ is OH, $H_2O$, halo, alkylcarboxy, nitrate, sulfate, or phosphate.

2. A compound according to claim 1 wherein $L_1$ and $L_2$ are the same ligand.

3. A compound according to claim 1 wherein $L_1$ and $L_2$ are different ligands.

4. A cis-diamine platinum (II) ascorbate compound having the formula:

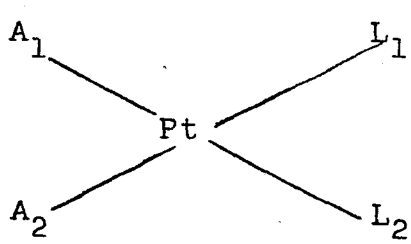

wherein Pt is in valence state II, $A_1$ and $A_2$ are ammonia or aliphatic amine ligands; or $A_1$ and $A_2$ are amine nitrogen atoms of a chelating diamine ligand; $L_1$ is the carbon atom of an ascorbate ligand; $L_2$ is the oxygen atom of an ascorbate ligand.

5. A compound according to claim 4 wherein $L_1$ and $L_2$ are from the same ascorbate ligand.

6. A compound according to claim 5 wherein $L_1$ is the C2 carbon of the ascorbate ligand and $L_2$ is the O5 oxygen atom of the ascorbate ligand.

7. A compound according to claim 4 wherein $L_1$ and $L_2$ are from different ascorbate ligands.

8. A compound according to any of claims 1 to 7 wherein $A_1$ and $A_2$ are ammonia.

9. A compound according to any of claims 1 to 7 wherein $A_1$ and $A_2$ are alkylamines.

10. A compound according to any of claims 1 to 7 wherein $A_1$ and $A_2$ are the amine nitrogen atoms of a chelating diamine ligand.

11. A compound according to claim 10 wherein said diamine ligand is a 1,2,diaminocycloalkane ligand.

12. A compound according to claim 11 wherein said 1,2,diaminocycloalkane is in the trans configuration.

13. A compound according to claim 11 wherein said 1,2,diaminocycloalkane is in the cis configuration.

14. A compound according to either of claims 12 or 13 wherein said 1,2,diaminocycloalkane is 1,2,diaminocyclohexane.

15. A compound according to any of claims 1 to 4 for use in therapy.

16. A method of preparing a compound according to any of claims 1 to 14, comprising reacting a platinum

complex of the formula:

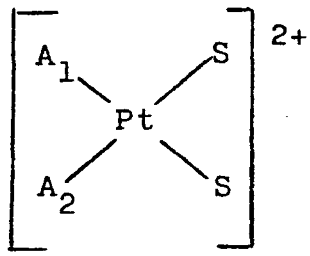

or

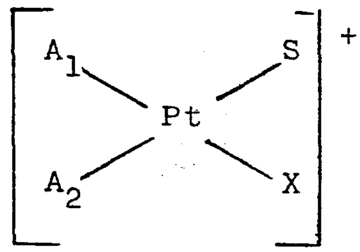

with a ligand or ligands $L_1$ and $L_2$ in an aqueous or alcoholic medium, wherein S is water or an alcohol, X is a halide and $A_1$, $A_2$, $L_1$ and $L_2$ are as defined in claim 1 or claim 4.

17. A method of preparing a pharmaceutical composition comprising mixing a compound according to any of claims 1 to 14 with a pharmaceutically acceptable diluent or carrier.

18. The use of a compound according to any of claims 1 to 14 in the manufacture of a medicament for treating an animal suffering from a malignant tumor.

FIG. 1 - IR SPECTRUM OF (Pt (CIS-DACH) (ASCORBATE) (ISOMER VI)

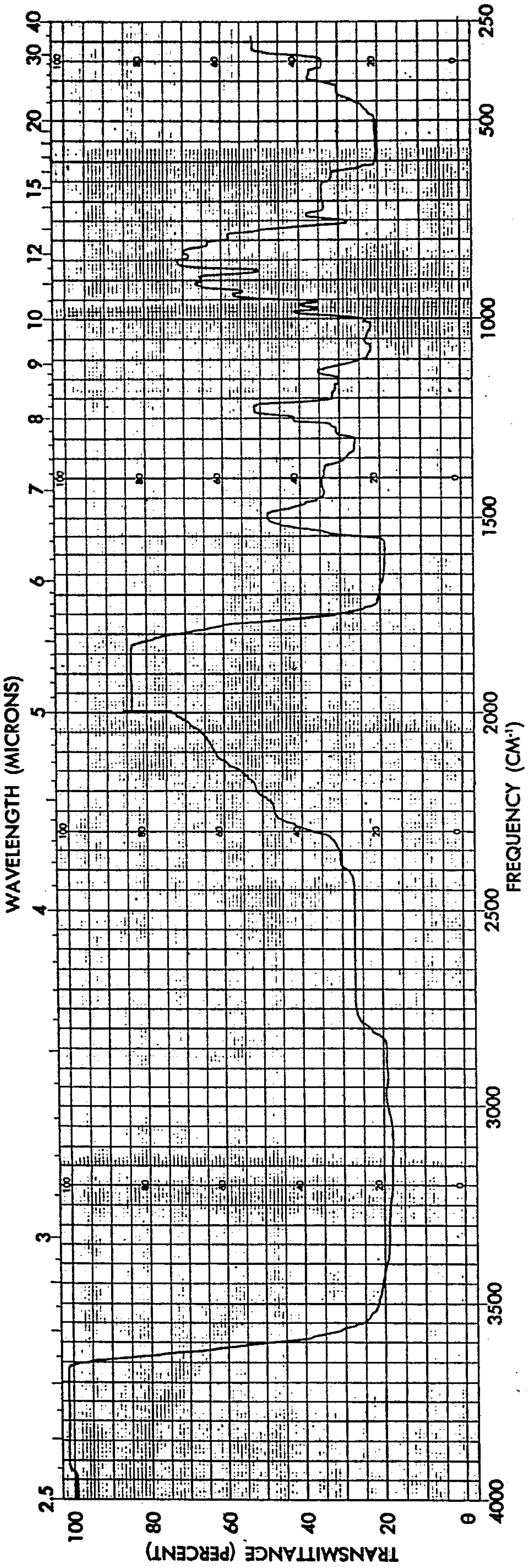

2/8

0174114

FIG. 2 - IR SPECTRUM OF (Pt. (CIS-DACH) (ASCORBATE) (ISOMER VII)

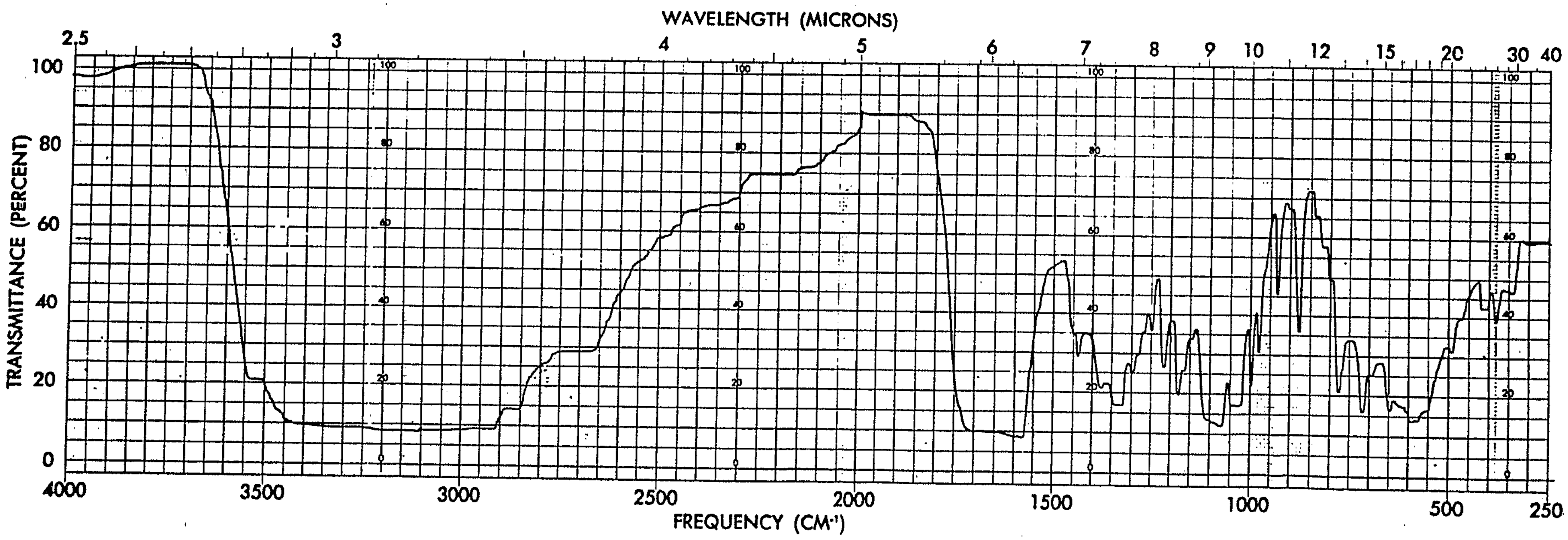

FIG. 3-IR SPECTRUM OF (Pt.(R,R-TRANS-DACH) (ASCORBATE) )

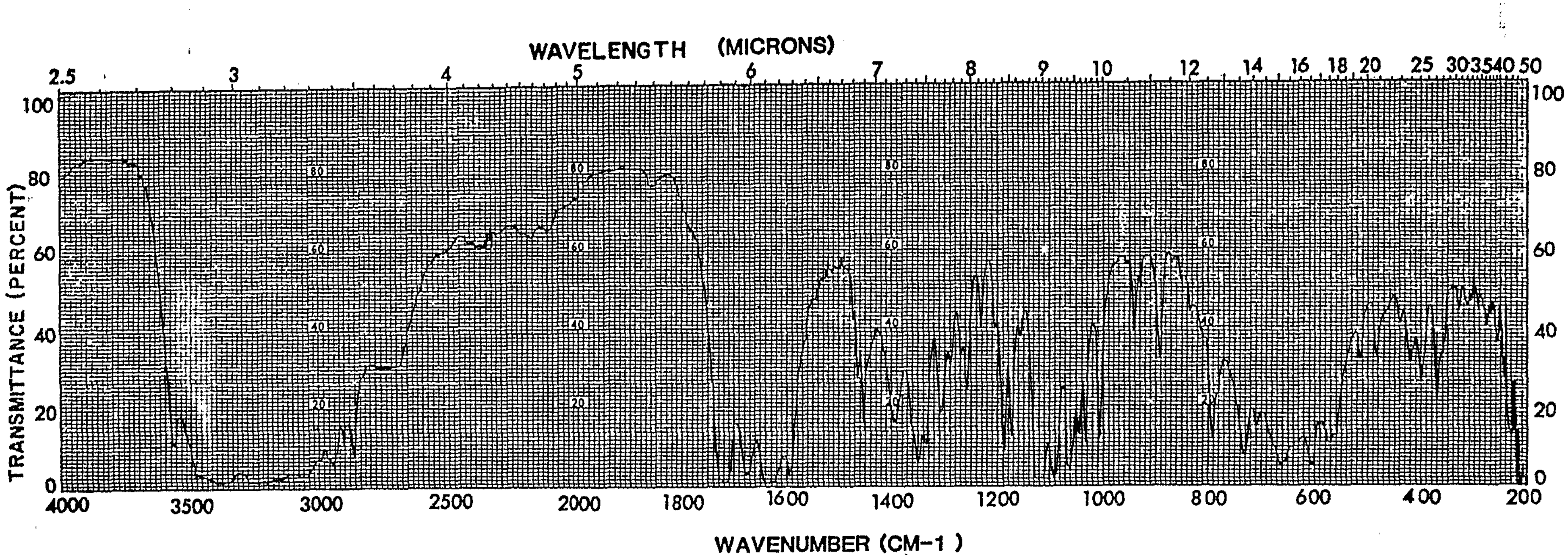

FIG. 4 - IR SPECTRUM OF (Pt (S,S-TRANS-DACH) (ASCORBATE) )

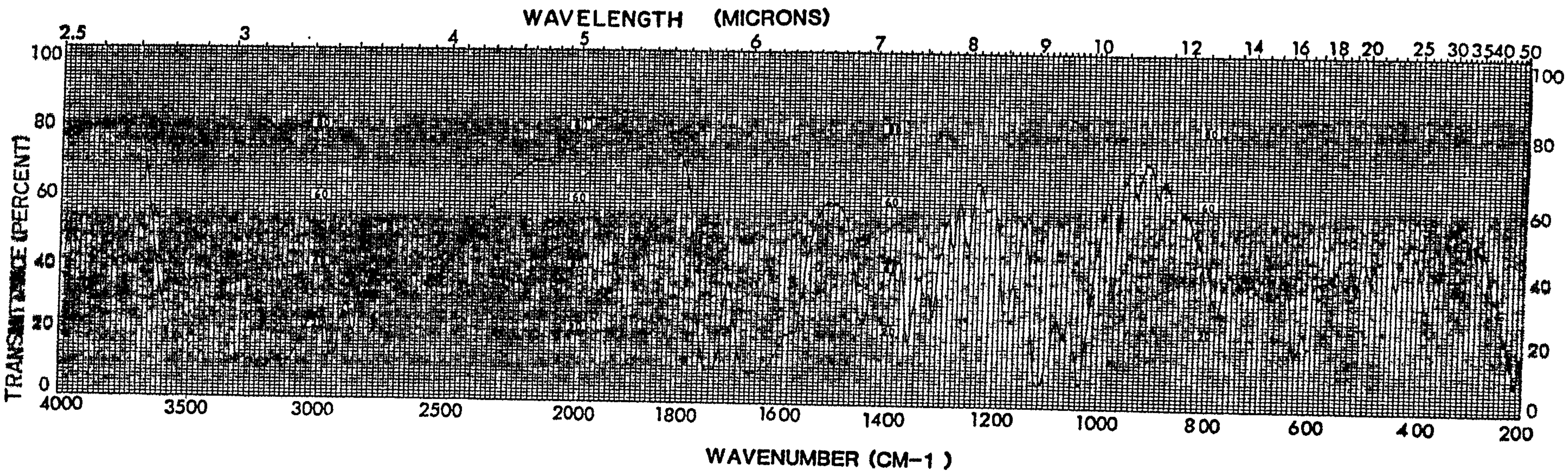

0174114

FIG. 5 - $^{13}C$ NMR SPECTRUM OF (Pt. (CIS-DACH) (ASCORBATE)$_2$)

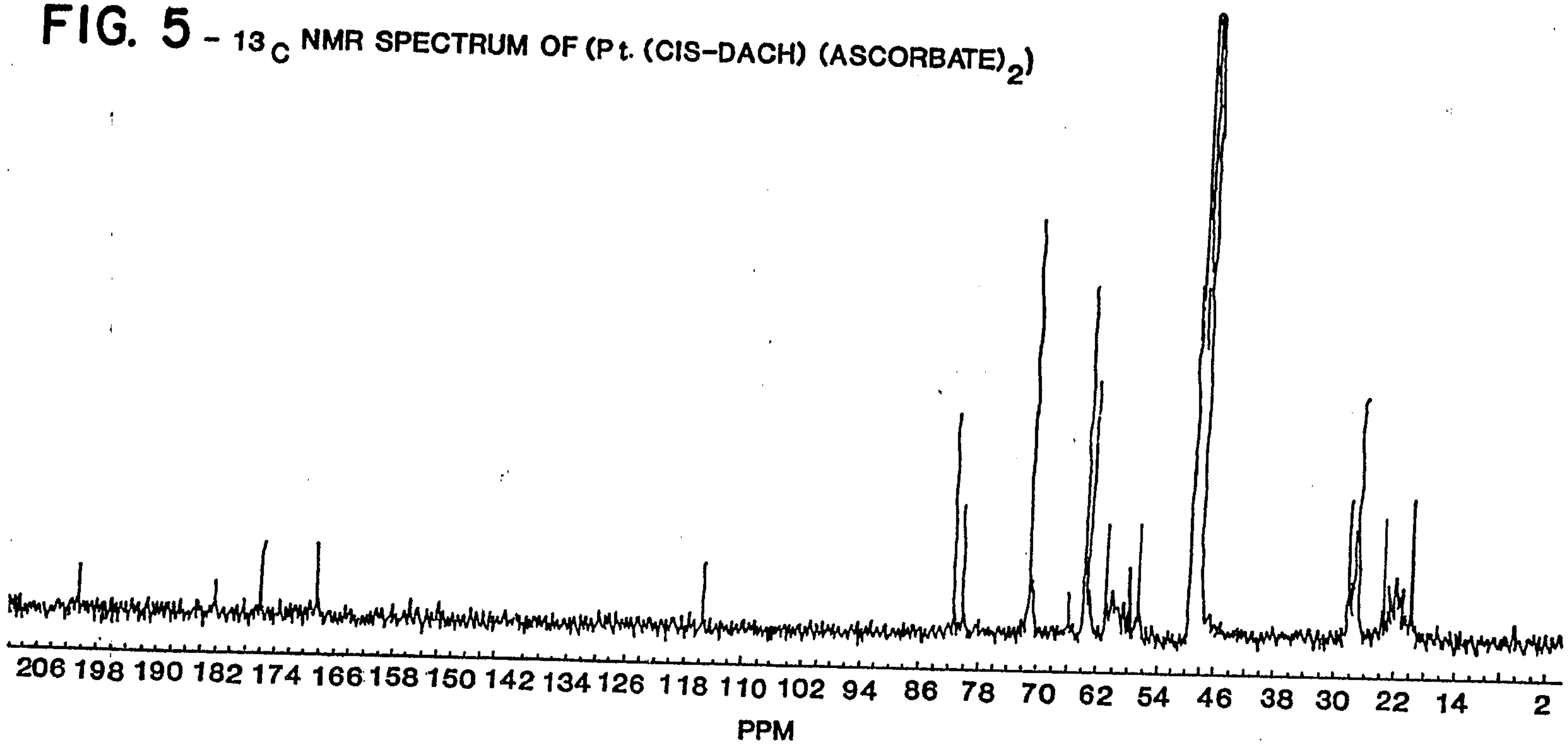

FIG. 6 - IR SPECTRUM OF (Pt (CIS-DACH) (ASCORBATE)$_2$)

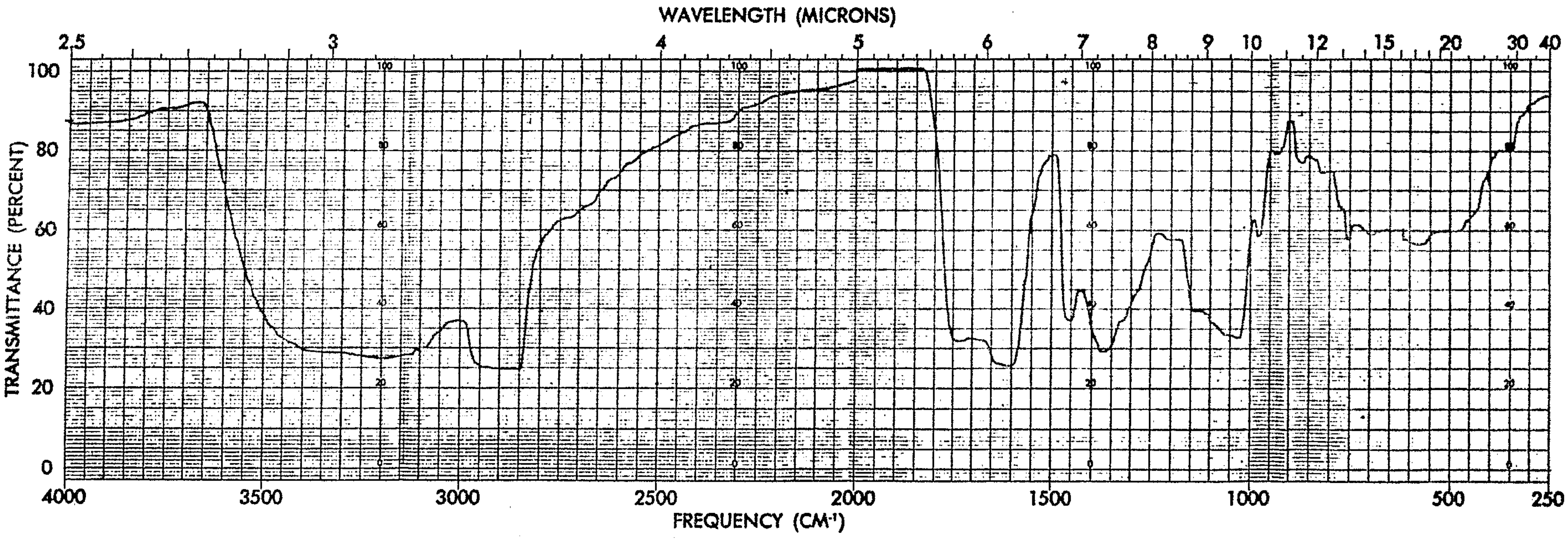

7/8

0174114

FIG. 7 - IR SPECTRUM OF CIS-(Pt (NH$_3$)$_2$ (ASCORBATE))

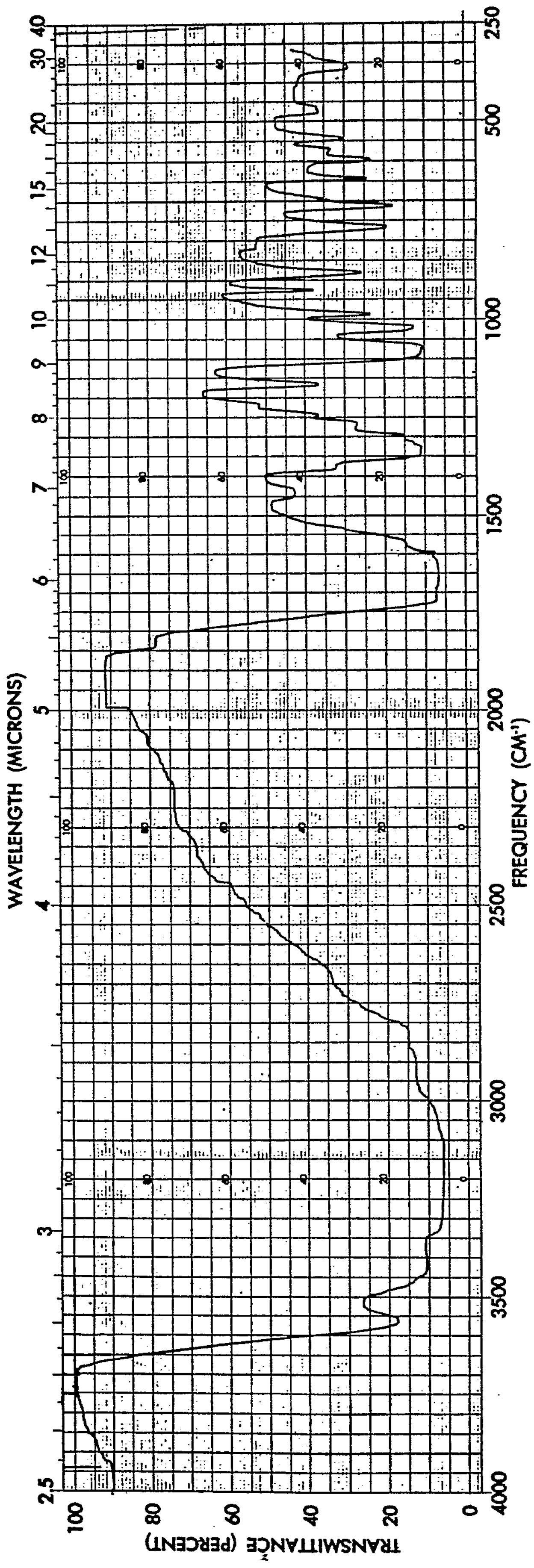

FIG. 8 - IR SPECTRUM OF (Pt(TRANS-DACH) (TETRAMETHYLREDUCTATE) )

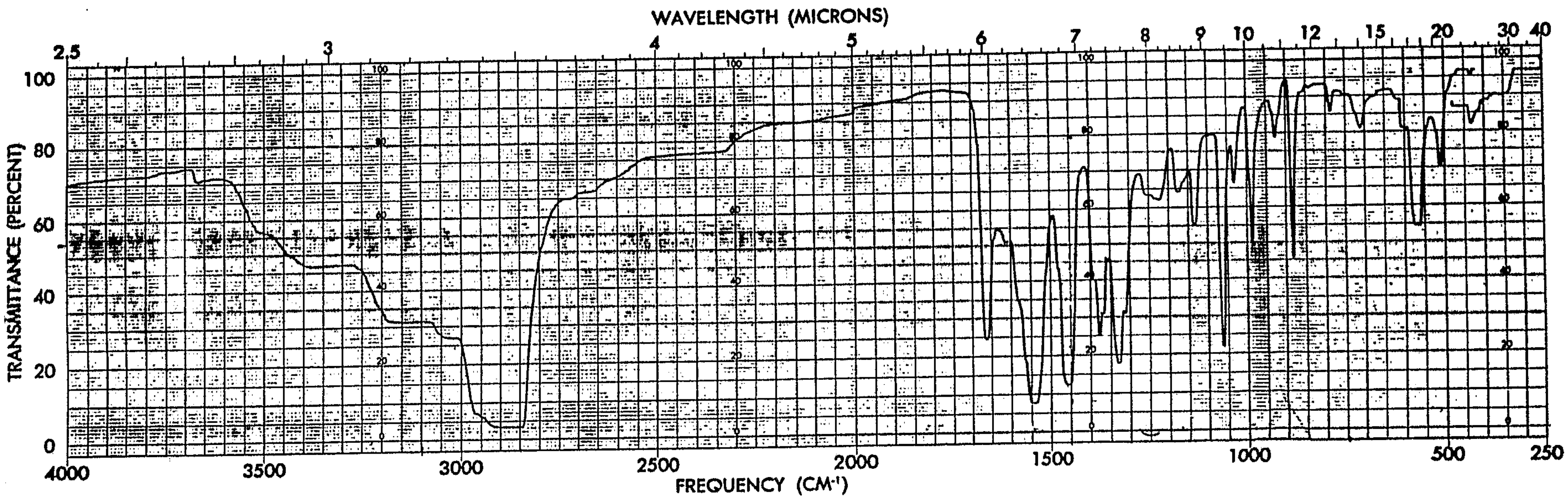

European Patent Office

# EUROPEAN SEARCH REPORT

0174114 Application number

EP 85 30 5731

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,P | EP-A-0 130 482 (RESEARCH CORPORATION) * claims 2,8,9 * | 1-18 | C 07 F 15/00<br>A 61 K 31/28 |
| A | EP-A-0 041 644 (BRISTOL-MYERS) * claims 1-7,11 * | 1 | |
| A | EP-A-0 008 936 (KIDANI) | 1 | |
| A,D | US-A-4 462 998 (ENGELHARD CORPORATION) | 1 | |
| A,D | US-A-4 457 926 (ENGELHARD CORPORATION) | 1 | |

TECHNICAL FIELDS SEARCHED (Int. Cl.4)

C 07 F 15/00

The present search report has been drawn up for all claims

| Place of search | Date of completion of the search | Examiner |
|---|---|---|
| BERLIN | 12-11-1985 | KAPTEYN H G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document